# EUROPEAN PATENT SPECIFICATION

(11) **EP 2 346 540 B1**
(45) Date of publication and mention of the grant of the patent: **03.04.2013**
(21) Application number: 09796797.0
(22) Date of filing: 06.11.2009
(51) Int. Cl.: A61L 27/14, A61L 27/36

(54) **MEDICAL DEVICE INCLUDING BACTERIAL CELLULOSE REINFORCED BY RESORBABLE REINFORCING MATERIALS**
MEDIZINISCHE VORRICHTUNG MIT BAKTERIELLER CELLULOSE, DIE MIT RESORBIERBAREN VERSTÄRKUNGSMATERIALIEN VERSTÄRKT IST
DISPOSITIF MÉDICAL COMPRENANT DE LA CELLULOSE BACTÉRIENNE AVEC RENFORT UTILISANT DES MATÉRIAUX DE RENFORT RÉSORBABLES

(30) Priority: 07.11.2008 US 112302 P
(43) Date of publication of application: 27.07.2011
(73) Proprietor: Sofradim Production, 01600 Trévoux (FR)
(72) Inventor: BAYON, Yves, F-69007 Lyon (FR); LADET, Sébastien, F-69006 Lyon (FR); LEFRANC, Olivier, F-01400 Chatillon sur Chalaronne (FR); GRAVAGNA, Philippe, F-69540 Irigny (FR)
(74) Representative: Maureau, Philippe
(86) International application number: PCT/IB2009/007680
(87) International publication number: WO 2010/052586

(56) References cited:
- EP-A1- 1 953 174
- WO-A1-2004/045458
- WO-A1-2007/106251
- WO-A2-2006/042287
- WO-A2-2008/079034
- US-A- 4 912 049
- US-A- 5 955 326

## Description

Medical devices in accordance with this disclosure include bacterial cellulose mechanically reinforced by bioresorbable materials. The present disclosure also relates to the use of the medical device for indications where the mechanical constraints are particularly high, for example, in the replacement of tendons and ligaments (referenced hereafter as "orthopedic soft tissues"), and when a permanent reinforcing member is preferred, for example, in the repair of a large hernia. In the present application, the terms "medical device" and "medical implant" have the same meaning. The present disclosure relates to a medical device comprising:
a reinforcing member; and
bacterial cellulose on at least a portion of the reinforcing member.
The reinforcing member comprises a bioresorbable material.
The bacterial cellulose is derived from *Acetobacter xylinum.* The bacterial cellulose is oxidized.
In embodiments, the reinforcing member is a textile made from multifilament yarns, monofilament yarns, or combinations thereof. The reinforcing member may be a non woven structure. The reinforcing member may be a combination of textiles, sheets and fibers.
The present disclosure also relates to a method for making a medical implant comprising:
culturing cellulose-producing bacteria in the presence of at least one reinforcing member thereby forming bacterial cellulose on at least a portion of the reinforcing member.
In embodiments, the bacteria is cultured in a culture vessel. The reinforcing member may be suspended at a distance of about 1 mm to about 3 mm above the bottom of the culture vessel during said culturing. Alternatively, the reinforcing member is disposed at the bottom of the culture vessel during said culturing. Alternatively, the reinforcing member may fixed at a distance of about 1 mm to about 3 mm above an adhesion surface of the bacteria during said culturing.

The present disclosure also relates to a method of treating a wound comprising contacting a wound with a medical device as described above.

In the present disclosure, the term "bioresorbable" is intended to mean the characteristic according to which an implant and/or a material is resorbed by the biological tissues and the surrounding fluids and disappears in vivo after a given period of time, that may vary, for example, from one day to several months, depending on the chemical nature of the implant and/or of the material.

In the present disclosure, the microbial cellulose is produced as wet pellicles or films from bacteria that synthesize cellulose. Cellulose is synthesized by bacteria belonging to the genera Acetobacter, Rhizobium, Agrobacterium, and Sarcina. Cellulose may be produced by certain bacteria from glucose in the presence of oxygen, (such as, for example, *Acetobacter xylinum,* referenced hereinafter as the "bacteria"), in static conditions or in a bioreactor (see, e.g. U.S. Patent Nos. 4,912,049 and 5,955,326, the entire disclosures of which are incorporated herein by this reference). Cellulose suitable for use in the present implants may be obtained by the fermentation of the bacteria. Oxidized cellulose resulting from the oxidation of the cellulose by periodic acid or nitrogen dioxide is employed.

Microbial cellulose possesses inherent characteristics which allow effective promotion of wound healing (see, e.g. U.S. Patent No. 7,390,492, the entire disclosure of which is incorporated herein by this reference). In this regard, microbial cellulose displays properties (such as a multi-layer three dimensional laminar structure) that distinguish it from plant cellulose and other natural polymeric materials. In this regard, microbial cellulose shows excellent wet strength, does not easily breakdown under compression and demonstrates high moisture handling ability.

In embodiments, cellulose pellicles or films are produced by culturing bacteria in culture vessels or bioreactors in the presence of reinforcing member. As the bacteria grow and proliferate, they form an extracellular network of cellulose on at least a portion of the reinforcing member. The reinforcing member is any bioresorbable material compatible with the culture conditions, allowing the growth of the bacteria and giving a desired mechanical strength to the device of the present disclosure after its final processing.

The reinforcing member is made from bioresorbable materials.

Suitable examples of bioresorbable materials may include but are not limited to, poly(lactic acid) (PLA), oxidized cellulose, polycaprolactone (PCL), polydioxanone (PDO), trimethylene carbonate (TMC), polyvinyl alcohol (PVA), polyhydroxyalkanoates (PHAs), polyamides, polyethers, copolymers thereof and combinations thereof. The bioresorbable materials may also be obtained from derivatives of polysaccharides among hyaluronic acid, alginic acid, poly(glucuronic acid), chitosan, chitin, cellulose, cross-linked derivatives thereof, and combinations thereof.

The bioresorbable materials may be selected from their ability to stand the culture conditions, in an aqueous medium, at a temperature of from about 10 °C to about 40 °C, during several days, at a mild acid pH in a range of about 2 to about 6. The bioresorbable materials may be selected from their ability to stand the full product processing, including the depyrogenation step as described in, for example, US Patent Publication No. 2007/0128243. The process may be optimized by lowering the temperature and the sodium hydroxide concentration.

In embodiments, the reinforcing member may be a textile which is formed using known techniques such as weaving, braiding or knitting. The textile may be made from multifilament yarns, monofilament yarns, or combinations thereof. The textile may be a two dimensional textile or a three dimensional textile, for example including for the latter spacers, giving controllable thickness to the textile. The yarns may be made from bioresorbable materials, non bioresorbable materials, or combinations thereof such as those identified above.

In embodiments, the reinforcing member may be a sheet, having a structure that is open enough to let the bacteria migrate through the sheet and encapsulate at least a portion of the sheet with the produced cellulose. The sheet may include pores, open to at least at one side of the sheet. In embodiments, the size of the pores may be from about 1 µm to about 10 mm, in embodiments, from about 50 µm to about 5 mm, in other embodiments, from about 500 µm to about 3 mm. In embodiments, the size of the pores may include two or more of these ranges. The softness of the sheet should be in the same order of the tissues to be replaced, such as for example, orthopaedic tissue or abdominal wall tissue. The sheet may be made from bioresorbable reinforcing materials, non bioresorbable materials, or combinations thereof, such as those identified above.

The reinforcing members may be of any shape.

The reinforcing members may have a thickness ranging from about 0.1 mm to 30 mm, in embodiments from about 0.5 mm to 5 mm.

In other embodiments, more than one reinforcing member or more than one type of reinforcing member may be incorporated into the present device.

The bacteria may be grown in the presence of the reinforcing member, either in static culture conditions or in a bioreactor as described in U.S. Patent Nos. 4,912,049 or 5,955,326. In static culture conditions of the bacteria, the reinforcing member may be laid at the bottom of the culture vessels or at a distance of about 1 mm to about 3 mm above the bottom of the vessels, fixed in position by any appropriate means. The reinforcing member may be fixed in such a way as to give a desired shape to reinforcing member and thereby imparting the desired shape to the cellulose pellicles or films. For example, in embodiments where the reinforcing members are textiles, they may be fixed with enough tension to impart a substantially flat orientation that is parallel to the bottom of the vessels.

When the bacteria are grown in bioreactors, the reinforcing members may be fixed at the surface where the bacteria will adhere and proliferate or at a distance of about 1 mm to about 3 mm above the adhesion surface of the bacteria. The reinforcing members may be fixed in the bioreactor as described hereinabove. For example, in the case of rotating disk bioreactor, the reinforcing members may be laid and fixed on the disks or fixed at a distance of about 1 mm to about 3 mm above the disks. The fixation of the reinforcing members should withstand the shear stress induced by the bioreactor at work on the discs.

In embodiments, a non woven reinforcing member may be formed from fibers of bioresorbable materials, non bioresorbable materials, and combinations thereof, may be embedded in a cellulose pellicle or film obtained by the culture of bacteria. In embodiments, the fibers may be mixed with the culture medium of the bacteria at a predetermined density to allow the formation of the non woven reinforcing member. Suitable densities include but are not limited to from about 0.1 to about 100 grams of fiber per liter of culture medium, in embodiments from about 1 to about 20 grams of fiber per liter of culture medium. In embodiments, the fibers may be added in the culture medium, several hours to a couple of days, after the seeding of the bacteria, in static culture conditions or in bioreactors.

In other embodiments, the bacteria may be grown on a combination, of reinforcing members, including textiles, sheets and fibers.

The replacement or repair of orthopaedic soft tissues may be achieved by the medical device of the present disclosure, which provides sufficient mechanical strength for such repairs.

The implant device has a final thickness from 1 mm to 20 mm, in embodiments, from 2 mm to 10 mm.

In embodiments, the final device may be asymmetric in its physical properties. Thus, the device according to the present disclosure may have a mechanical strength, at least in one direction, measured according to ISO standard 13934-1 (properties of substances in tensile testing), from about 250 N to about 1000 N, in embodiments, from about 250 N to about 500 N.

The medical device of the present disclosure may have an elongation from about 1% to about 10% measured at 100 N in the more mechanically resistant direction, measured according to ISO standard 13934-1.

In embodiments, the present reinforced cellulose pellicles or films may be kept wet with a solution, dispersion cellulose concentration or suspension having from about 1 % to about 20 %. The cellulose pellicles or films are dried by freeze-drying.

In embodiments, the present reinforced cellulose pellicles or films may be compressed using known techniques so as to provide the desired final dimensions and cellulose density.

In embodiments, the present reinforced cellulose pellicles or films are cross-linked. In embodiments, chemical functionalization of the cellulose, provides a desired degradation profile and other features of the final device.

The medical implants in accordance with this disclosure may be produced at a predetermined size or produced in large sheets which may be cut to sizes appropriate for the envisaged application. The medical implants may be packaged in single or dual pouches and sterilized using conventional techniques, such as, but not limited to, irradiation with beta (electronic irradiation) or gamma (irradiation using radioactive cobalt) rays at about 25 KGy to about 35 KGy, and/or sterilized by ethylene oxide. In embodiments where hydrolytically unstable materials are used in forming the implant, such as polyglycolic acid or polylactic acid, the composites are packaged under sufficiently dry conditions to ensure that no degradation of the composite takes place during storage.

The present medical devices including bacterial cellulose mechanically reinforced by bioresorbable or non bioresorbable materials obtained by fermentation of *Acetobacter xylinum,* may advantageously maintain one or more of the unique properties of bacterial cellulose (such as, for example, high biocompatibility, extreme hydrophilicity, unique multi-layered three dimensional laminar structures which provide its moisture handling properties, excellent wet strength, high resistance to breakdown under compression, conformability, absence of generation of harmful particles of the cellulose mesh after rubbing against surrounding tissues or erosion at sharp edges of tissues - e.g. sharp edges of bone and cartilage tissues) with minimal processing, in particular to keep the shape of the pellicle or film.

The present medical devices may be easily fixed into its desired final shape with the reinforcing member in one step, avoiding the inclusion of reinforcing member by handling the cellulose, and potentially by modifying its unique properties as obtained after the fermentation process.

The medical devices of the present disclosure may be used for challenging surgical conditions where the mechanical constraints are particularly high such as the repair and/or replacement of orthopedic soft tissues.

## Claims

1. A medical implant comprising:
a reinforcing member; and
bacterial cellulose on at least a portion of the reinforcing member,
wherein the reinforcing member comprises a bioresorbable material, the reinforcing member being a textile made from multifilament yarns, monofilament yarns, or combinations thereof, the medical device having a thickness from 1 mm to 20 mm,
wherein the bacterial cellulose is oxidized and is under the form of a freeze-dried pellicle or film.

2. The medical implant of claim 1, wherein the bacterial cellulose is derived from *Acetobacter xylinum.*

3. The medical implant of any one of claims 1-2, wherein the reinforcing member is a non woven structure.

4. The medical implant of any one of claims 1-3, wherein the reinforcing member is a combination of textiles, sheets and fibers.

5. A method for making a medical implant comprising:
culturing cellulose-producing bacteria in the presence of at least one reinforcing member thereby forming bacterial cellulose pellicle or film on at least a portion of the reinforcing member, wherein the reinforcing member comprises a bioresorbable material, the reinforcing member being a textile made from multifilament yarns, monofilament yarns, or combinations thereof,
wherein the bacteria is cultured in a culture vessel, the reinforcing member is fixed at a distance of 1 mm to 3 mm above an adhesion surface of the bacteria during said culturing,
wherein the cellulose is oxidized, and
wherein the cellulose pellicle or film is freeze-dried.

## Patentansprüche

1. Medizinisches Implantat, umfassend:
ein Verstärkungselement; und
bakterielle Cellulose auf zumindest einem Teil des Verstärkungselements,
wobei das Verstärkungselement ein bioresorbierbares Material umfasst, wobei das Verstärkungselement ein Textil ist, das aus Multifilamentgarnen, Monofilamentgarnen oder Kombinationen davon hergestellt ist, wobei die medizinische Vorrichtung eine Dicke von 1 mm bis 20 mm aufweist,
wobei die bakterielle Cellulose oxidiert ist und in Form eines gefriergetrockneten Pellikels oder Films vorliegt.

2. Medizinisches Implantat nach Anspruch 1, wobei die bakterielle Cellulose von *Acetobacterxylinum* abgeleitet ist.

3. Medizinisches Implantat nach einem der Ansprüche 1 bis 2, wobei das Verstärkungselement eine Vliesstruktur ist.

4. Medizinisches Implantat nach einem der Ansprüche 1 bis 3, wobei das Verstärkungselement eine Kombination aus Textilien, Tüchern und Fasern ist.

5. Verfahren zum Herstellen eines medizinischen Implantats, umfassend:
Züchten von celluloseproduzierenden Bakterien in Gegenwart zumindest eines Verstärkungselements, um ein Pellikel oder einen Film bakterieller Cellulose auf zumindest einem Teil des Verstärkungselements zu bilden, wobei das Verstärkungselement ein bioresorbierbares Material umfasst, wobei das Verstärkungselement ein Textil ist, das aus Multifilamentgarnen, Monofilamentgarnen oder Kombinationen davon hergestellt ist,
wobei die Bakterien in einem Kulturgefäß gezüchtet werden, wobei das Verstärkungselement während des Züchtens in einem Abstand von 1 mm bis 3 mm über einer Haftfläche der Bakterien befestigt ist,
wobei die Cellulose oxidiert wird, und
wobei das Cellulosepellikel oder der Cellulosefilm gefriergetrocknet werden.

## Revendications

1. Implant médical comprenant :
un élément de renfort ; et
de la cellulose bactérienne sur au moins une partie de l'élément de renfort,
où l'élément de renfort comprend un matériau biorésorbable, l'élément de renfort étant un textile fabriqué à partir de fils multifilaments, de fils monofilaments, ou de combinaisons de ceux-ci, le dispositif médical ayant une épaisseur comprise entre 1 mm et 20 mm,
où la cellulose bactérienne est oxydée et est sous la forme d'une pellicule ou d'un film lyophilisé(e).

2. Implant médical selon la revendication 1, dans lequel la cellulose bactérienne est dérivée *d'Acetobacterxylinum.*

3. Implant médical selon l'une quelconque des revendications 1-2, dans lequel l'élément de renfort est une structure non tissée.

4. Implant médical selon l'une quelconque des revendications 1-3, dans lequel l'élément de renfort est une combinaison de textiles, de feuilles et de fibres.

5. Procédé pour fabriquer un implant médical, comprenant l'étape de cultiver des bactéries produisant de la cellulose en présence d'au moins un élément de renfort, en formant ainsi une pellicule ou un film de cellulose bactérienne sur au moins une partie de l'élément de renfort, où l'élément de renfort comprend un matériau biorésorbable, l'élément de renfort étant un textile fabriqué à partir de fils multifilaments, de fils monofilaments, ou de combinaisons de ceux-ci,
où les bactéries sont cultivées dans un récipient de culture, l'élément de renfort est fixé à une distance de 1 mm à 3 mm au-dessus d'une surface d'adhésion des bactéries pendant ladite culture,
où la cellulose est oxydée, et
où la pellicule ou le film de cellulose est lyophilisé(e).
